(19) Europäisches Patentamt / European Patent Office / Office européen des brevets

(11) **EP 1 868 724 B1**

(12) **EUROPEAN PATENT SPECIFICATION**

(45) Date of publication and mention of the grant of the patent:
**13.03.2013 Bulletin 2013/11**

(21) Application number: **06723962.4**

(22) Date of filing: **03.04.2006**

(51) Int Cl.:
***B01L 7/00*** *(2006.01)*

(86) International application number:
**PCT/EP2006/003003**

(87) International publication number:
**WO 2006/105919 (12.10.2006 Gazette 2006/41)**

(54) **THERMOCYCLING OF A BLOCK COMPRISING MULTIPLE SAMPLE**

THERMOZYKLIERUNG EINES MEHRERE PROBEN ENTHALTENDEN BLOCKS

CYCLE THERMIQUE D UN BLOC COMPRENANT DES ECHANTILLONS MULTIPLES

(84) Designated Contracting States:
**AT BE BG CH CY CZ DE DK EE ES FI FR GB GR HU IE IS IT LI LT LU LV MC NL PL PT RO SE SI SK TR**

(30) Priority: **04.04.2005 EP 05007267**

(43) Date of publication of application:
**26.12.2007 Bulletin 2007/52**

(60) Divisional application:
**12168865.9 / 2 495 046**

(73) Proprietors:
- **Roche Diagnostics GmbH**
  **68305 Mannheim (DE)**
  Designated Contracting States:
  **DE**
- **F. Hoffmann-La Roche AG**
  **4070 Basel (CH)**
  Designated Contracting States:
  **AT BE BG CH CY CZ DK EE ES FI FR GB GR HU IE IS IT LI LT LU LV MC NL PL PT RO SE SI SK TR**

(72) Inventors:
- **SCHLAUBITZ, Thomas**
  **6045 Meggen (CH)**
- **BURDACK, Torsten**
  **80687 Muenchen (DE)**
- **FEDERER, Paul**
  **6110 Wolhusen (CH)**
- **GEORGE, Christian**
  **82515 Wolfratshausen (DE)**
- **GRUETER, Guido**
  **CH-6038 Gisikon (CH)**
- **SCHOLLE, Andreas**
  **86899 Landsberg am Lech (DE)**
- **TENZLER, Guenter**
  **80638 Muenchen (DE)**

(74) Representative: **Herren, Barbara**
**Roche Diagnostics AG**
**Patent Department**
**Forrenstrasse**
**6343 Rotkreuz (CH)**

(56) References cited:

| | |
|---|---|
| **WO-A-01/24930** | **WO-A-2004/024330** |
| **WO-A-2004/105947** | **US-A- 4 950 608** |
| **US-B1- 6 633 785** | |

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 868 724 B1

Printed by Jouve, 75001 PARIS (FR)

## Description

### Field of invention

The present invention relates to the field of high throughput analysis of samples. In particular, the present invention is directed to a device, a system and a method for simultaneous tempering of multiple samples.

### Prior art background

Devices for tempering samples or reaction mixtures in a controlled way are used in almost all fields of chemistry or biochemistry, whereas basic science is affected in the same manner than industrial development or pharmaceutical production. Since labor time as well as reagents are expensive, development is tending to increase the throughput of production and analysis, while at the same time, to minimize the necessary reaction volumes.

In general tempering devices have a thermal block that is in thermal contact with the sample under investigation. The thermal block is tempered to a desired temperature affecting the temperature of the sample, too. The simplest thermal block is a common boilerplate.

In order to realize an efficient tempering, the device should have means to heat and cool the samples. For this purpose, the thermal block can be connected with two separate means or with a single means able to perform both heating and cooling. Such a single tempering means is e.g. a flow-through means, whereas a pipe system within or close to the thermal block is streamed with an externally tempered fluid, e.g. water or oil, transporting heat to or from the thermal block. In case of two separate means, in general a resistive heating in combination with a dissipative cooling is utilized. A good summary about thermal management in the field of medical and laboratory equipment is written by Robert Smythe (Medical Device & Diagnostic Industry Magazine, Jan. 1998, p. 151-157) and the following is an excerpt of this article.

A common dissipative cooling device is a heat sink in combination with a fan. Generally, heat sinks are made from aluminum because of the metal's relatively high thermal conductivity and low cost. They are extruded, stamped, bonded, cast, or machined to achieve a shape that will maximize surface area, facilitating the absorption of heat by the surrounding cooler air. Most have a fin or pin design. When used with fans (forced convection), heat sinks can dissipate large amounts of heat while keeping the targeted components at 10 - 15 °C above ambient temperature. Heat sinks are inexpensive and offer installation flexibility but cannot cool components at or below ambient temperature. Also, heat sinks do not permit temperature control.

More sophisticated setups utilize thermoelectric devices (TEC) as heat pumps for heating and active cooling of a thermal block. Thermoelectric devices are solid-state heat pumps made from semiconductor materials comprising a series of p-type and n-type semiconductor pairs or junctions sandwiched between ceramic plates. Heat is absorbed by electrons at the cold junction as they pass from a low energy level in a p-type element to a higher energy level in an n-type element. At the hot junction, energy is expelled to e.g. a heat sink as the electrons move from the high-energy n-type element to a low-energy p-type element. A dc power supply provides the energy to move the electrons through the system. A typical TEC will contain up to 127 junctions and can pump as much as 120 W of heat. The amount of heat pumped is proportional to the amount of current flowing through the TEC and therefore, tight temperature control is possible. By reversing the current, TECs can function as heaters or coolers, which can be useful in controlling an object in changing ambient environments or in cycling at different temperatures. Sizes range from 2 to 62 mm, and multiple TECs can be used for greater cooling. Because of the relatively large amount of heat being pumped over a small area, TECs in general require a heat sink to dissipate the heat into the ambient environment. A well known type of TECs is the Peltier elements.

The dissipation of heat is essential for efficient cooling. If the heat can not be dissipated at its origin, said heat can be transferred to another place using heat pipes. A heat pipe is a sealed vacuum vessel with an inner wick structure that transfers heat by the evaporation and condensation of an internal working fluid. Ammonia, water, acetone, or methanol are typically used, although special fluids are used for cryogenic and high-temperature applications. As heat is absorbed at one side of the heat pipe, the working fluid is vaporized, creating a pressure gradient within the heat pipe. The vapor is forced to flow to the cooler end of the pipe, where it condenses, giving up its latent heat to the wick structure and than to the ambient environment via e.g. a heat sink. The condensed working fluid returns to the evaporator via gravity or capillary action within the inner wick structure. Because heat pipes exploit the latent heat effect of the working fluid, they can be designed to keep a component near ambient conditions. Though they are most elective when the condensed fluid is working with gravity, heat pipes can work in any orientation. Heat pipes are typically small and highly reliable, but they can not cool objects below ambient temperature.

A thermal block can be tempered with two heat pipes, whereas one heat pipe transports heat from a heat source to said thermal block and whereas the other heat pipe transports heat away from said thermal block. A thermal block with two heat pipes is disclosed in WO 01/51209. A plurality of heat pipes are used in US 4,950,608 to realize a temperature regulating container. A heat pipe with a controllable thermal conductance is disclosed in US 4,387,762.

Besides the heat pipes, the evaporated solid state enclosure with a liquid-vapor equilibrium in form of a pipe, these solid state enclosure are also known in a platelike form produced by the company Thermacore (Lancester, USA), called Therma-Base™. These Therma-Base™ have a substantially planar shape and are used e.g. in computers to distribute heat generated at integrated circuits (US 6,256,199). An apparatus for temperature regulation of elements in thermal contact with a fluid contained in liquid-vapor equilibrium inside an enclosure is disclosed in US 5,161,609. US 5,819,842 describes a temperature control unit comprising a spreader plate for the independent control of multiple samples which are in close proximity.

WO01/24930 discloses a thermocycler assembly with a plate shaped heat pipe arranged between a heat pump and a thermal block to obtain a highly homogenous heat distribution within the heat block. WO 2004/105947 discloses a thermocycler assembly with a segmented thermal block, wherein the segments can be cycled independently, and heat pipes arranged to obtain a homogenous heat distribution within individual segments of the heat block.

Thus, it was the object of the present invention to provide a device for the simultaneous tempering of samples. In one aspect of the present invention, the invention relates to simultaneous thermocycling of multiple samples to perform PCR in a microtiter plate format.

**<u>Brief description of the invention</u>**

The invention is directed to a device to temper a plurality of individual samples in a parallel manner. More precisely, the invention is directed to a device suitable to perform a plurality of simultaneous PCR amplification within multiple samples.

One subject matter of the present invention is a device for the simultaneous thermocycling of multiple samples comprising:

a) a thermal base 8 formed like a thermal block comprising said multiple samples,

b) at least one heat pump 2,

c) a thermal base 4,

d) a heat sink 5 and

e) a control unit 3 to control said simultaneous thermocycling of multiple samples,

characterized in that the thermal base (4) is arranged between and is in thermal contact with said heat sink (5) and said at least one heat pump (2), said at least one heat pump (2) is in thermal contact with said thermal base (8), wherein said thermal base (4) is a vapor chamber device for transporting and distributing heat and wherein said thermal contact is based on a paste having a high thermal conductance, a thermally conductive foil or mechanical force.

Another subject matter of the present invention is a device for the simultaneous thermocycling of multiple samples comprising:

a) a thermal block 1 comprising said multiple samples,
b) at least one heat pump 2,
c) a thermal base 4,
d) a heat sink 5 and
e) a control unit 3 to control said simultaneous thermocycling of multiple samples,

characterized in that the thermal base (4) is arranged between and is in thermal contact with said heat sink (5) and said at least one heat pump (2), said at least one heat pump (2) is in thermal contact with said thermal block (1), wherein said thermal base (4) is a vapor chamber device for transporting and distributing heat and wherein said thermal contact is based on a paste having a high thermal conductance, a thermally conductive foil or mechanical force, wherein the cross section area of said thermal base (4) is larger than the cross section area of said thermal block (1) by at least a factor of 1.5 and said thermal base (4) has the same cross section area as said heat sink (2), said cross section areas are in parallel to the respective contact areas.

Throughout the present invention, the simultaneous thermocycling of multiple samples comprises all kinds of tempering of a plurality of samples. Simultaneous thermocycling summarizes a cyclic variation of the temperature of said multiple samples, whereas the temperature at the beginning of one cycle is the same.

The phrase multiple samples comprises any number of samples, whereas said multiple samples can be arrange in several ways. A common way to arrange multiple samples is the use of microtiter plates. Alternatively, multiple reaction vessels may be arranged in a holding means. Within the scope of the present invention, the multiple samples are fluid samples. Each of said multiple samples comprises a solvent and one or more solved targets to be analyzed.

A thermal block 1 is a solid state device disposed to have a good thermal conductance. There is a plurality of materials known to someone skilled in the art that have a good thermal conductance and without being bound to theory, most materials having a good electrical conductance are good thermal conductors, too. Therefore, materials like copper, aluminum, silver or graphite are suitable for the thermal block. On the other hand, also plastics and ceramics may have sufficient thermal conductance to be used as material for the thermal block.

A heat pump 2 is an active device that is able to transport heat. In general heat pumps are so-called thermoelectric devices (TEC) made from semiconductor materials that need electricity to work. A dc power supply provides the energy for heating and cooling, whereas reversing the current does reverse the direction of heat being pumped. A well known type of TECs are the Peltier elements.

A thermal base 4 is a vapor chamber device for transporting and distributing heat. Throughout the present invention a thermal base is a special heat pipe, whereas said thermal base has regions of substantially planar shape. The term heat pipe is an established name for a sealed vacuum vessel with an inner wick structure that transfers heat by the evaporation and condensation of an internal working fluid. As heat is absorbed at one side of the heat pipe, the working fluid is vaporized, creating a pressure gradient within said heat pipe. The vapor is forced to flow to the cooler end of the heat pipe, where it condenses, giving up its latent heat to the ambient environment. The condensed working fluid returns to the evaporator via gravity or capillary action within the inner wick structure. A thermal base in general is a passive device, but it can be designed as an active device, too, if said thermal base is equipped with control means. Said control means modify the thermal conductivity of the thermal base by adjusting either the flow rate within the enclosure or the volume of the enclosure affecting the vacuum within the vessel.

A heat sink 5 is a device to dissipate heat. In general, a heat sink is made out of a thermally conductive material analogous to the thermal block outlined before. Therefore, heat sinks are mostly made out of metal, preferably out of aluminum or copper. Another suitable material for heat sinks is graphite. Alternatively, heat sinks may be formed out of plastics and ceramics, if only a good thermal conductance is realized. In order to realize a maximum dissipation of heat, heat sinks are disposed to provide a large surface-to-volume ratio. This is realized by an assembly of fins arranged on a base plate. A large surface-to-volume ratio reduces the heat transfer resistance between the heat sink and the surrounding air.

A control unit 3 is a device to control said simultaneous thermocycling of multiple samples. Within the present invention said control unit adjusts the power supply of the heat pumps, modifying the amount of heat transported to or transported from the thermal block. Additionally, the control unit may operate the optional control means of the thermal bases.

Yet another aspect of this invention is a method for the simultaneous thermocycling of multiple samples comprising the steps

a) providing a thermal base 8 formed like a thermal block with multiple recesses, at east one heat pump **2,** a thermal base **4,** a heat sink 5 and a control unit **3,**

b) arranging said thermal base 8 with multiple recesses, said at least one heat pump **2,** said thermal base **4,** and said heat sink **5,** wherein

- said heat sink **5** is in thermal contact with said thermal base **4,**
- said first thermal base **4** is in thermal contact with said at least one heat pump **2** and
- said at least one heat pump **2** is either in thermal contact with said thermal base 8;

c) placing said multiple samples within the recesses of said thermal base 8 and

d) performing a thermocycling protocol with said control unit 3,

wherein said thermal contact is based on either a paste having a high thermal conductance, a thermally conductive foil or mechanical force, wherein said thermal base (4), (8) is a vapor chamber device for transporting and distributing heat.

## <u>Detailed description of the invention</u>

One subject matter of the present invention is a device for the simultaneous thermocycling of multiple samples comprising:

a) a thermal base 8 formed like a thermal block comprising said multiple samples,

b) at least one heat pump **2,**

c) a thermal base **4,**

d) a heat sink **5** and

e) a control unit **3** to control said simultaneous thermocycling of multiple samples,

characterized in that the thermal base (4) is arranged between and is in thermal contact with said heat sink (5) and said at least one heat pump (2), said at least one heat pump (2) is in thermal contact with said thermal base (8), wherein said thermal base (4) is a vapor chamber device for transporting and distributing heat and wherein said thermal contact is based on a paste having a high thermal conductance, a thermally conductive foil or mechanical force.

Another subject matter of the present invention is a device for the simultaneous thermocycling of multiple samples comprising:

a) a thermal block 1 comprising said multiple samples,
b) at least one heat pump 2,
c) a thermal base 4,
d) a heat sink 5 and
e) a control unit 3 to control said simultaneous thermocycling of multiple samples,

characterized in that the thermal base (4) is arranged between and is in thermal contact with said heat sink (5) and said at least one heat pump (2), said at least one heat pump (2) is in thermal contact with said thermal base (8), wherein said thermal base (4) is a vapor chamber device for transporting and distributing heat and wherein said thermal contact is based on a paste having a high thermal conductance, a thermally conductive foil or mechanical force.

There are a large number of devices known to a person skilled in the art that are able to temper a sample in a cyclic fashion. The phrase thermocycling summarizes a cyclic variation of the temperature of a sample, whereas the temperature at the beginning of one cycle is the same as the temperature at the end of said cycle. One temperature cycle comprises phases of heating, cooling (temperature ramps) and phases of constant temperature. The temperature variation with time is summarized by the phrase "thermocycling protocol".

If the device should be able to simultaneously temper an assembly of multiple samples, e.g. the wells of a microliter plate, and the results of the experiments within the multiple samples should be comparable, one has to guarantee that the thermocycling of the samples in the center of the assembly and at the boarder of the assembly are preferably identical. Moreover, it is desirable to perform the temperature ramps of the thermocycling protocol as fast as possible, but without overshooting the temperatures of the multiple samples when reaching the phases of constant temperature.

In a preferred embodiment of the device according to the present invention, said thermal block 1 is made out of a thermally conductive material.

Thermally conductive materials are materials that have a good thermal conductivity and low heat capacity. In heat transfer analysis the ratio of thermal conductivity and heat capacity is also defined as thermal diffusivity

$$\alpha = k/(\rho \cdot c_p)$$

where k is the thermal conductivity, measured in W/(m'K), $\rho$'$c_p$ is the volumetric heat capacity, measured in J/($m^3 \cdot K$). The SI unit of the thermal diffusivity is $m^2/s$.

Substances with high thermal diffusivities rapidly adjust their temperatures to that of their surroundings, because they conduct heat quickly in comparison to their thermal bulk. Thermally diffusive materials are materials having a good thermal conductivity, and without being bound to theory, most materials having a good electrical conductance also have a good thermal diffusivity, too.

On the other hand, although they have much smaller thermal diffusivities, there are also some plastics, ceramics and polymers that have sufficient thermal properties for the present invention. Plastics have thermal diffusivities of up to $\alpha = 0.2 \cdot 10^{-6}$ $m^2/s$, ceramics of up to $\alpha = 0.4 \cdot 10^{-6}$ $m^2/s$. Polymeric materials e.g. can have thermal conductivities of up to $k = 10$ $Wm^{-1}K^{-1}$.

In a more preferred embodiment of the device according to the present invention, said thermal block 1 is made out of metal, preferably out of aluminum or silver.

There is a plurality of metallic materials known to someone skilled in the art that have a good thermal conductance and that are suitable for the thermal block, e.g. copper, aluminum or silver. Copper for example has a thermal diffusivity of about $\alpha = 107 \cdot 10^{-6}$ $m^2/s$, silver of about $\alpha = 166 \cdot 10^{-6}$ $m^2/s$, whereas aluminum has about of about $\alpha = 93 \cdot 10^{-6}$ $m^2/s$ (all at 300 K). Nevertheless, aluminum is a preferred

material, because it is cheap and easy to process. Note that in the majority of cases the metallic materials are not pure but alloys, whereas the thermal conductance of the material will be depending on the composition of said alloy.

In general, the thermal block 1 of the present invention is a cuboid with a top-view cross section area A, a length 1, a width w and a height h, having the preferred dimensions of 1 = 5 - 200 mm, w = 5 - 200 mm and h = 3 - 100 mm.

In another preferred embodiment of the device according to the present invention, said thermal block **1** comprises recesses 7 disposed to receive said multiple samples.

In this embodiment of the device according to the present invention, the thermal block 1 is equipped with multiple recesses 7, whereas said recesses 7 are arranged on the top side reaching to the inside of said thermal block **1**. It is preferred that said recesses have all the same size. Said recesses 7 may be obtained by drilling in a homogeneous thermal block 1. Alternatively, said drilling in a homogeneous thermal block **1** may be performed in such a way that the recesses 7 form holes crossing the entire height of the thermal block 1. Besides the method of drilling in a homogeneous thermal block **1**, other methods like molding, electroforming, deep drawing or electrical discharge machining may be used to manufacture the thermal block with recesses.

In a further preferred embodiment of the device according to the present invention, said multiple samples are placed directly in said recesses 7 of the thermal block **1** or via reaction vessels each comprising one of said multiple samples.

The recesses 7 are disposed to receive said multiple samples, whereas several possibilities are applicable within the scope of the present invention. In one embodiment, the multiple samples are positioned in said recesses 7 directly via e.g. a pipetting step. If necessary, the recesses 7 may be coated with a material that is inert for the samples and that is cleanable to recycle the thermal block **1** for further

In one embodiment of the present invention, the thermal block is replaced by a special thermal base 8 that is formed like a thermal block itself.

In an also preferred embodiment of the device according to the present invention, said reaction vessels are linked to form one or more groups, preferably said reaction vessels are linked to form a multiwell plate.

Each of said multiple recesses 7 can receive a separate reaction vessel or one or more groups of linked reaction vessels can be positioned in said multiple recesses 7. A well-known single reaction vessel suitable for the present invention is e.g. an Eppendorf cup, whereas a suitable group of linked reaction vessels is e.g. an Eppendorf cup strip or a microtiter plate having e.g. 96, 384 or 1536 individual wells.

In yet another preferred embodiment of the device according to the present invention, said at least one heat pump 2 is a thermoelectric device, preferably a semiconductor device, more preferably a Peltier element.

A heat pump 2 is an active element that needs electricity to generate and/or transport heat and that is also named thermoelectric devices (TEC) in literature. In general, TEC heat pumps 2 are solid-state heat pumps made from semiconductor materials comprising a series of p-type and n-type semiconductor pairs or junctions sandwiched between ceramic plates. A dc power supply provides the energy to move the electrons through the system thereby transporting heat. A typical TEC will contain up to 127 junctions and can pump as much as 120 W of heat, whereas the amount of heat pumped is proportional to the amount of current flowing through the TEC. Therefore, TECs offer a tight temperature control. By reversing the current, TECs can function as heaters or coolers, which can be useful in controlling an object in changing ambient environments or in cycling at different temperatures. A well known type of TECs are the Peltier elements. Such Peltier elements are commercially available in several different versions with respect to performance, shape and materials. TECs are rectangular or round, they may have centered bore holes for fixation and exist in different heights. Special TECs are optimized to stand extensive switching between the working modes and are applicable of up to 150 °C. In general, the semiconductor device is sandwiched between to ceramic plates. These ceramic plates may be equipped with slits to reduce thermal stress. In order to counter the bimetallic effect, the ceramic plates may be covered partly with a metallic material (e.g. copper).

In another preferred variant of the device according to the present invention, said thermal base 4 is a heat conducting device comprising a liquid-vapor equilibrium within a solid state enclosure.

As mentioned before, a thermal base 4 within the scope of the present invention is basically analogous to the heat pipes known to someone skilled in the art, with the difference that the thermal base 4 has at least partially a plate like structure in comparison to the pipe like structure of the heat pipes. In general, heat pipes as well as thermal bases are solid state enclosures with an inner wick structure that transfers heat by the evaporation and condensation of an internal working fluid. In other words, within the sealed vessel a liquid-vapor equilibrium of the internal working fluid persists, whereas the local equilibrium is depending on the local temperature. In more detail, if heat is absorbed at one side of the heat pipe, the working fluid is vaporized, creating a pressure gradient within the heat pipe. The vapor is forced to flow to the cooler end of the pipe, where it condenses, giving up its latent heat to the ambient environment. The condensed working fluid returns to the evaporator via gravity or capillary action within the inner wick structure. Ammonia, water, acetone, or methanol are typically used as work fluids, although special fluids are used for cryogenic and high-temperature applications.

The thermal base has a very high quasi thermal conductivity of up to $2 \cdot 10^5$ $Wm^{-1}K^{-1}$ and therefore, the spreading of heat across the entire cross section area of the thermal base is very efficient. This on the one hand, increases the homogeneity during the heating process and on the other hand, decrease the required time for the cooling process, because the heat transfer resistance of the heat sink will be further reduced.

A preferred variant of the device according to the present invention comprises a thermal base 4 that is substantially planar.

In a more preferred embodiment of the device according to the present invention said thermal base 4 is free of recesses.

Within the scope of the present invention it is preferred that the thermal base 4 is substantially planar, whereas substantially planar summarizes cuboid thermal bases 4 with a top-view cross section area A, a length 1, a width w and a height h, having the preferred dimensions of 1= 10 - 500 mm, w = 10 - 500 mm and h = 3 -15 mm.

Throughout the present invention the phrase "free of recesses" is used to emphasize that in certain preferred embodiments of the present invention the thermal base has a continuous top-view cross section area A that is uninterrupted by recesses. In other words, a thermal base that is free of recesses has a planar surface at least in the area of thermal contact with the neighboring device parts.

Within the scope of the present invention the phrase "thermal contact" between two components is used to emphasize that the physical contact between two components has to be optimized towards a high thermal conductance. In other words, throughout the present invention a "thermal contact" is an optimized "physical contact" to improve the thermal conductance between two components. Since air is a poor thermal conductor, one has to guarantee that the amount of air between two components in thermal contact is as small as possible. There are several possibilities to minimize air in the contact zone of two solid state materials, whereas these possibilities can be classified in two groups, namely a direct thermal contact and an indirect thermal contact.

One variant of indirect thermal contact utilizes a paste having a high thermal conductance as linker between the two components, e.g. thermal grease. In another variant of indirect thermal contact preferably a soft, thermally conductive foil, e.g. a graphite foil is used as an interface material between the two components. Such a graphite foil can even a certain roughness of the components and reduces the mechanical stress due to thermal expansion.

On the other hand, it is preferred to apply a mechanical force such that a direct thermal contact is sufficient and no additional interface materials between the two components are needed. It is also preferred that both contact areas are as flat as possible to minimize the air gap between the components. Note that it is of advantage to apply a mechanical force to press together the two components even for the embodiments with indirect thermal contact, because this can further improve the thermal conductance.

In a more preferred variant of the device according to the present invention, said thermal base 4 is in thermal contact with said heat sink 5 and via a graphite foil with said at least one heat pump 2, whereas said at least one heat pump 2 is in thermal contact with said thermal block 1 via a graphite foil as well. If desired, thermal grease may be used as an additional interface material between the thermal base 4 and said heat sink 5.

In yet another preferred variant of the device according to the present invention, said at least one heat pump 2 is used to generate heat and to transport said heat to said thermal block 1.

In a more preferred embodiment of the device according to the present invention, said at least one heat pump 2 is further used for the active transport of heat from said thermal block 1 to said thermal base 4.

By reversing the current, TECs can function either as heaters or as coolers. In the one operation mode the TEC generates heat and said heat is transported to one of the two ceramic plates of the device. In the other operation mode the TEC transports heat from one of the ceramic plate to the other ceramic plate of the device and therefore, actively cools one of the ceramic plates. In other words, while one of the sides of the TEC will be cooled, the other side of the TEC will be heated.

In an also preferred embodiment of the device according to the present invention, the cross section area of said thermal base 4 is by less than 20 % larger or smaller than the cross section area of said heat sink 5 and the cross section area of said thermal base 4 is larger than the cross section area of said thermal block 1, said cross section areas are in parallel to the respective contact areas.

During the cooling of the thermal block a large amount of heat has to be dissipated in a short time. If the amount of heat that needs to be dissipated becomes even larger, at first glance this can be encountered by using simply a larger heat sink 5 that accordingly provides a larger surface area for dissipation. This assumption is only correct to some extent, because by using a common metal heat sink 5 with its restricted thermal conductivity only a certain fraction of the surface area close to the heat source will participate in the dissipative process. Therefore, enlarging the cross section area of a common metal heat sink 5 alone is no appropriate way to handle the dissipation of large amounts of heat. Within the present invention, the cross section area is always the cross section area of the device components in top-view. Note that the schematic pictures of several embodiments of the device in Figure 1 represent side-views of the composition.

Using a thermal base 4 in combination with a heat sink 5 in accordance with the present invention improves the heat dissipation, because the enormous thermal conductance of the thermal base 4 assures that even a heat sink 5 being much larger than the heat source will participate effectively in the dissipative process. The optimization of the dissipative process helps to reduce the required time for the cooling steps within the thermocycling protocol.

In another more preferred variant of the device according to the present invention, said cross section area of said thermal base 4 is larger than the cross section area of said thermal block 1 by at least a factor of 1.5, preferably by at least a factor of 4 and said thermal base 4 has the same cross section area as said heat sink 2, said cross section areas are in parallel to the respective contact areas.

The maximal reasonable ratio of the cross section area of said thermal base 4 and the cross section area of said thermal block 1 is depending on the thermal conductance of the thermal base 4. The same is true for the cross section area ratio of the heat sink 5 and the thermal base 4. Providing a heat sink 5 with a cross section area much larger than that of the said thermal base 4 does not further improve the heat dissipation.

In another more preferred variant of the device according to the present invention, said thermal base is provided with control means 9 to vary the heat conducting properties of said thermal base 4.

It is preferred to provide the thermal base with a control means 9, because if the heat conducting properties of said thermal base may be varied, the influence of said thermal bases may be switched "on" and "off" as desired by the different procedures of the thermocycling protocol. For example, it is desirable to minimize the heat conducting properties of the thermal base 4 for the heating procedure of the thermocycling protocol. If the thermal base 4 can not be switched "off" during the heating procedure, a larger portion of the heat generated at the at least one heat pump 2 will be dissipated immediately at the heat sink 5.

There are several ways to control the heat conducting properties of a thermal base (see e.g. US 5,417,686). In general, the heat conducting properties of a thermal base are depending on the liquid-vapor equilibrium of the internal working fluid affected by the vessel vacuum as well as on the transportation of gas and liquid within the sealed vessel.

A more preferred embodiment according to the present invention is a device, wherein said control means 9 vary the heat conducting properties of a thermal base by changing the volume within said thermal base.

Another more preferred embodiment according to the present invention is a device, wherein said control means 9 vary the heat conducting properties of a thermal base by changing the flow rate within said thermal base.

The liquid-vapor equilibrium of the internal working fluid within a thermal base can be modified by changing the volume of the thermal base. This can be done by providing an additional vessel connected to said thermal base via an opening, whereas the volume of said additional vessel is adjustable. Said additional vessel can be e.g. a syringe or a bellows. Alternatively, the vacuum within said thermal base can be adjusted directly by using a vacuum pump connected to an opening of the vessel. Moreover, the heat conducting properties of the thermal base can be modified by affecting the flow rate within said vessel. Here, a throttling valve is suitable that may be operated from the outside without affecting the vacuum within the vessel that divides the thermal base into compartments.

In a preferred variant of the device according to the present invention, said heat sink 5 is made out of a thermally conductive material.

In a more preferred variant of the device according to the present invention, said heat sink 5 is made out of metal, preferably out of aluminum, cooper, silver or graphite.

Concerning the thermally conductive material of the heat sink 5, the same statements are valid as addressed before with respect to the thermal block 1.

In yet another preferred variant of the device according to the present invention, said heat sink 5 is disposed to provide a maximized surface-to-volume ratio.

Without being bound to theory, the amount of heat that can be dissipated by said heat sink 5 is directly proportional to its surface area. Therefore, it is desirable to provide a heat sink with an optimized surface-to-volume ratio, because of the limited amount of space within the device of the present invention.

In a more preferred variant of the device according to the present invention, said large surface-to-volume ratio is provided by an assembly of fins arranged on a base plate.

Also preferred is a device according to the present invention, wherein said heat sink 5 is cooled by air or by water flow.

An interstitial assembly of fins provides a large surface area, whereas the solid base plate represents the thermal contact area with the thermal base 4. The heat sink 5 dissipates heat to the surrounding. Since this dissipative process is most effective for large temperature differences between the surrounding atmosphere and the heat sink 5, it is desirable to actively cool the surrounding. This can be done either by air flow produced by a fan or by liquid flow produced by e.g. a peristaltic pump.

In yet another preferred variant of the device according to the present invention, said control unit 3 controls the properties of said at least one heat pump 2.

In a more preferred variant of the device according to the present invention, said control unit 3 further controls said control means 9 to vary the heat conducting properties of said thermal base 4.

The device according to the present invention is equipped with a control unit 3. Said control unit 3 is an electric device, e.g. a computer, that controls the power supply of the at least one heat pump 2 and therefore, adjusts their heating or The device according to the present invention is equipped with a control unit 3. Said control unit 3 is an electric device, e.g. a computer, that controls the power supply of the at least one heat pump 2 and therefore, adjusts their heating or cooling properties. Additionally, said control unit 3 can operate the control means 9 of the thermal base.

Another preferred embodiment according to the present invention is a device, wherein said thermocycling is performed to realize nucleic acid amplifications within said multiple samples.

A more preferred embodiment according to the present invention is a device further comprising a means to monitor said nucleic acid amplifications in real-time.

Within the scope of the present invention all nucleic acid amplifications known to someone skilled in the art are applicable, e.g. the polymerase chain reaction (PCR) the Ligase Chain Reaction (LCR), Polymerase Ligase Chain Reaction, Gap-LCR, Repair Chain Reaction, 3SR, strand displacement amplification (SDA), transcription mediated amplification (TMA) or Qβ-amplification.

In general, nucleic acid amplifications are monitored in real-time using fluorescence dyes known to someone skilled in the art. To measure the fluorescence signals all kinds of optical means are suitable within the scope of the present invention. Preferred are CCD cameras or photometers that may be utilized with and without additional optical components like lenses, optical filters or folding mirrors.

If a certain application requires that the optical means has to be oriented below the thermal block 1, e.g. to monitor the fluorescence intensity of the multiple samples through bottom holes in said thermal block 1, it is possible to arrange the composition out of a heat sink 5, a thermal base 4, the heat pumps 2 sideways of said thermal block 1. To gain a homogeneous thermocycling of the thermal block 1, it is possible to arrange one of said compositions at each of the four sides of said thermal block 1. Alternatively, a single composition may be arranged surrounding the thermal block 1.

In yet another preferred variant of the device according to the present invention, said thermal base 4 is substantially planar.

In a more preferred variant of the device according to the present invention, said thermal base 4 is substantially planar being in thermal contact with said heat sink 5.

As mentioned before, substantially planar summarizes cuboid thermal bases with a top-view cross section area A, a length 1, a width w and a height h and said thermal base 4 has the preferred dimensions of 1=10 - 500 mm, w = 10 - 500 *mm* and h = 3 - 15 mm. With respect to the thermal contact all possibilities mentioned before are applicable for this preferred variant, too.

It is preferred that there is an additional interface material between said heat pumps 2 and said thermal bases 4. A preferred interface material is a graphite foil as outlined before.

In another more preferred variant of the device according to the present invention, the cross section area of said thermal base 4 is by less than 20 % larger or smaller as the cross section area of said heat sink 5, the cross section area of said thermal base 4 is larger than the cross section area of said thermal base 8 said cross section areas are in parallel to the respective contact areas.

The positive effect of a heat sink **5** as well as a thermal base **4** that have both a larger cross section area as the thermal block **1** was discussed in detail before. In brief, using a thermal base **4** in combination with a heat sink **5** improves the heat dissipation, because the enormous thermal conductance of the thermal base **4** assures that even a heat sink **5** being much larger than the heat source will participate effectively in the dissipative process.

In an even more preferred variant of the device according to the present invention, the cross section area of said thermal base 4 is larger than the cross section area of said thermal block 1 by at least a factor of 1.5, preferably by at least a factor of 4 and said thermal base 4 has the same cross section area as said heat sink 2, said cross section areas are in parallel to the respective contact areas.

The reasonable cross section area ratios of said thermal base 4 and said thermal block 1 as well as of said thermal base 4 and said heat sink 2 are depending on the thermal conductance of the thermal base 4.

As mentioned before, when TECs are used as heat pumps, reversing the current of these thermoelectric elements provides either a heating or a cooling device.

Another preferred embodiment according to the present invention is a device, wherein said thermal base **4** and/or said thermal base 8 are provided with control means **9** to vary the heat conducting properties of said thermal bases **4**,**8.**

It is preferred to provide each thermal base with a control means **9**, because if the heat conducting properties of said thermal bases may be varied independently, the influence of said thermal bases may be switched "on" and "off" as desired by the different procedures of the thermocycling protocoL For example, in an embodiment with a thermal base 4 and a thermal base **8**, it is desirable to minimize the heat conducting properties of the thermal base **4** and to maximize the heat conducting properties of the thermal base 8 for the heating procedure of the thermocycling protocol. If the thermal base **4** can not be switched "off" during the heating procedure, a larger portion of the heat generated at the at least one heat pump **2** will be dissipated immediately at the heat sink **5**.

Note that the ways to control the heat conducting properties of a thermal base as well as the embodiments of heat sink **5**, heat pump **2**, thermal block **1**, control means **9** and control unit **3** as described before are also applicable with respect to the device with thermal base **4** and thermal base **8**.

In a more preferred variant of the device according to the present invention, said control unit **3** further controls said control means **9** to vary the heat conducting properties of said thermal bases **4**,**8**.

The device according to the present invention is equipped with a control unit **3**. Said control unit **3** is an electric device, e.g. a computer, that controls the power supply of the at least one heat pump **2** and therefore, adjusts their heating or cooling properties. Additionally, said control unit **3** can operate the control means **9** of the at least one thermal base **4**,**8.**

Another aspect of this invention is a method for the simultaneous thermocycling of multiple samples comprising the steps

a) providing a thermal base **8** with multiple recesses, at least one heat pump **2**, a thermal base **4**, , a heat sink **5** and a control unit **3**,

b) arranging said thermal base 8 with multiple recesses, said at least one heat pump **2**, said thermal base **4**, and said heat sink **5**, wherein

- said heat sink **5** is in thermal contact with said thermal base **4**,
- said thermal base **4** is in thermal contact with said at least one heat pump **2** and
- said at least one heat pump **2** is in thermal contact with said thermal base **8,**

c) placing said multiple samples within the recesses of said thermal base **8** and

d) performing a thermocycling protocol with said control unit 3,

wherein said thermal contact is based on either a paste having a high thermal conductance, a thermally conductive foil or mechanical force, wherein said thermal base (4), (8) is a vapor chamber device for transporting and distributing heat

The phrase thermocycling protocol summarizes a cyclic variation of the temperature of a sample, whereas the temperature at the beginning of one cycle is the same as the temperature at the end of said cycle. One temperature cycle comprises phases of heating, cooling (temperature ramps) and phases of constant temperature.

As mentioned before, the phrase "thermal contact" between two components is used throughout the present invention to emphasize that the contact has to be optimized towards a high thermal conductance. The thermal contact can be optimized e.g. by a paste, e.g. thermal grease, having a high thermal conductance as linker between the two components or by a soft, thermally conductive foil, e.g. a graphite foil as an intermediate between two components. In all cases it is of advantage, if the two components are pressed together by mechanical force.

In a preferred variant of the method according to the present invention, said thermal base **4** is in thermal contact with said heat sink **5** and via a graphite foil with said at least one heat pump **2**, whereas said at least one heat pump **2** is in thermal contact with said thermal base **8.**

In another preferred variant of the method according to the present invention, said base **4** is substantially planar.

In a more preferred variant of the method according to the present invention, said thermal base **4** is free of recesses.

In yet another preferred variant of the method according to the present invention, the cross section area of said thermal base **4** is by less than 20 % larger or smaller than the cross section area of said heat sink **5** and the cross section area of said thermal base **4** is larger than the cross section area of said thermal base **8** said cross section areas are in parallel to the respective contact areas.

In a more preferred variant of the method according to the present invention, said cross section area of said thermal base **4** is larger than the cross section area of said thermal base **8** by at least a factor of 1.5, preferably by at least a factor of **4** and said thermal base **4** has the same cross section area as said heat sink **2**, said cross section areas are in parallel to the respective contact areas.

The reasons for the above indicated preferred arrangements were already discussed before with respect to the device according to the present invention.

In yet another preferred embodiment of the method according to the present invention, said multiple samples are placed within said recesses of said thermal block **1** directly or via reaction vessels each comprising one of said multiple samples.

In a more preferred embodiment of the method according to the present invention, said reaction vessels are linked to form one or more groups, preferably said reaction vessels are linked to form of a multiwell plate.

The different options to place the multiple samples in the thermal base **8** were already discussed before with respect to the device according to the present invention.

In a further preferred embodiment of the method according to the present invention, said thermocycling protocol is suitable to perform nucleic acid amplifications within said multiple samples.

Even more preferred is a method according to the present invention, wherein said nucleic acid amplifications are monitored in real-time.

Yet another embodiment is a system for the simultaneous thermocycling of multiple samples in order to perform multiple nucleic acid amplification reactions comprising

a) a device according to the present invention and
b) reagents necessary to perform said multiple nucleic acid amplification reactions.

Reagents throughout this application are all kinds of chemicals that are necessary to perform one of the methods outlined above with the aid of the inventive device according to the present invention. These reagents may be liquids or solids, pure materials or mixtures, they may be provided 'ready-to-use' or as concentrates.

In an embodiment said reagents comprise buffer solutions, detergents, enzymes, nucleotides and primers.

The reagents of this embodiment are the reagents necessary to perform PCR amplifications. In more detail, reagents are a set of single nucleotides, a polymerase, a pair of primers and buffer solutions.

In another embodiment said multiple nucleic acid amplification reactions are multiple PCR amplifications that are monitored in real-time.

Description of the Figures

Figure 1 Schematic pictures of several embodiments of the device according to the present invention.
Figure 2 Heat pictures of the thermal block during a heating procedure of the thermal block.
Figure 3 Heat pictures of the thermal block during a cooling procedure of the thermal block.
Figure 4 Graph illustrating several temperatures associated with the thermal block as a function of time during the term of a thermocycling protocol comprising 6 cycles.
Figure 5 Detailed graph illustrating several temperatures associated with the thermal block as a function of time during the term of one thermocycling cycle.
Figure 6 Real-time amplification curves of a Parvovirus B19 fragment. Five different target concentrations were analysed by real-time PCR and each concentration is represented by five different wells of the plate. (a: $10^6$ copies; b: $10^5$ copies; c: $10^4$ copies; d: $10^3$ copies; e: $10^2$ copies).
Figure 7 Real-time amplification curves of a Parvovirus B19 fragment. 96 real-time amplification curves recorder in 96 different wells of a plate, each containing $10^4$ copies of the target sequence.

Example 1

A device according to the present invention for the thermocycling of a 384 multiwell plate comprises a homemade thermal block out of the aluminum alloy AlMgSi 0,5. An aluminum block with the dimension 109 x 73 x 9.1 mm was used to form 384 recesses by drilling, each conic recess has a top diameter of 3.44 mm (angle 17°) and a depth of 6.8 mm.

Below said thermal block 6 Peltier elements are arranged, whereas the thermal contact is enhanced via a thermal conductive graphite foil. The used Peltier elements are suitable for multiple thermocycling procedures and can heat up to 130 °C. Additionally, each of them has a cooling capacity of 75 W.

Via a second thermal conductive graphite foil, the 6 Peltier elements are arranged on a thermal base. The used thermal base is customized production of Thermacore™ and has the dimension of 248 x 198 x 5 mm. The vessel wall is made out of copper and the working fluid is water.

The used heat sink is commercially available from Webra (product number W-209) and is made out of the aluminum alloy AlMgSi 0,5 with the dimension 250 x 200 x 75 mm. Between the heat sink and the thermal base a commercial thermal grease is applied in order to enhance the thermal contact.

All four components of the device are fixed together by 17 screws and springs and the dissipative process is enhanced by four fans circulation air at the heat sink.

Example 2

Heat pictures of the thermal block of a device as described in Example 1 were recorded with an IR-camera (commercially available at the company FLIR) during a heating procedure (Figure 2) and a cooling procedure (Figure 3).

The heating procedure (Figure 2) started at a temperature of 55 °C with a heating rate of 4 °C/s until 95 °C were reached, whereas the cooling procedure (Figure 3) started at a temperature of 95 °C with a cooling rate of 2 °C/s until 55°C. were reached. The pictures were taking at different times during the heating procedure and cooling procedure, respectively.

Example 3

In Figure 4 different characteristic temperatures of 6 successive temperature cycles of the following thermocycling protocol are plotted as a function of time:

| step | temp | ramp | hold time | number |
|---|---|---|---|---|
| PreCycle | 40°C | 2.0°C/s | 120 sec | 1 |
| MainCycle | 95°C | 4.4°C/s | 10 sec | 6 |
| | 55°C | 2.0°C/s | 10 sec | |
| | 72°C | 4.4°C/s | 10 sec | |

7 different temperature profiles are included in the figure, the temperature profile of the thermocycling protocol ('Soll Temp'), the theoretical temperature of the thermal block ('Soll Ist'), the measured temperature of the thermal block ('Ist Temp'), the mean temperature measured within 9 recesses of the thermal block ('Mean'), the minimal measured temperature of said 9 recesses of the thermal block ('Min'), the maximal measured temperature of said 9 recesses of the thermal block ('Max') and the homogeneity of the 9 recess measurements ('Hom'; homogeneity = maximal recess temperature - minimal recess temperature).

A standard multiwell plate was arranged in the recesses of the thermal block and 9 wells distributed across the cross section of the thermal block were filled with oil (Type Applied Biosystems, Nujol Nineral Oil, Part No. 0186-2302). The temperature was measured using a thermocouple (Thermocouples Omega 5TC-TT-36-72) for each recess. The temperature of the thermal block was measured with an internal temperature sensor within the thermal block.

In Figure 5 a magnification of the last cycle of the sequence is plotted to illustrate the different profiles in more detail.

Example 4

To further demonstrate the validity of the invention, real-time PCR amplifications of different target concentrations with a detection based on fluorescent-dye labelled hybridisation probes were performed using the apparatus described in Example 1. As a test system the real-time PCR amplification of a 177 bp fragment of the Parvovirus B19 (SEQ ID NO:1) was chosen. As fluorescent probe the HybridisationProbe pair (SEQ ID NO:4 and SEQ ID NO:5) of the LightCycler - Parvovirus B19 Quantification Kit (Roche Applied Science, Article No. 3 246 809) or SybrGreen was used. Results are displayed in Figure 6 (HybridisationProbe pair) and Figure 7 (SybrGreen).

*PCR*

A partial fragment of the parvovirus B19 sequence was cloned into a pCR™ 2.1 plasmid vector (Invitrogen). Parvovirus B19 plasmid DNA dilutions were prepared in 10 mM Tris-HCl, pH 8.3. Per PCR reaction $10^6$ to 100 copies of the plasmid target were used for amplification.

For PCR amplification the LightCycler - Parvovirus B19 Quantification Kit (Roche Applied Science, Article No. 3 246 809) was used. A typical PCR assay consisted of $10^6$ to 100 copies of Parvovirus B19 plasmid, reaction buffer, detection buffer and 1 U of FastStart Taq DNA polymerase according to manufacturer's instructions. The PCR protocol consisted of an initial denaturation step at 95 °C for 10 min, followed by 40 cycles of amplification at 95 °C for 10 s, 60 °C for 15 s and 72 °C for 10 s. Ramp rates were 4.8 °C for heating and 2.4 °C for cooling, respectively. PCR reactions were run in a total volume of 20 μl in a white 384-well microtiter plate (custom-made product of Treff, Switzerland).

Fluorescence emission was detected in each cycle at the end of the annealing step at 60 °C using a CCD camera coupled to an optical system comprising a telecentric lens in order to measure the fluorescence signals of all wells of the plate simultaneously. The used optical system is described in the European patent application EP 05000863.0 (filed January 18, 2005). The HybridisationProbe pair was excited at 480 nm, whereas emission was measured at 640 nm. SybrGreen was excited at 470 nm, whereas emission was measured at 530 nm. Exposure time was set to 1000 ms.

In Figure 6 amplification curves of 5 different target concentrations are plotted, whereas each target concentration is represented by 5 different wells (distributed across the 384 well plate). The groups of amplification curves based on the same target concentration are labelled with (a) $10^6$ copies (medium $C_p$ (elbow value) 16.6; SD 0.033), (b)

$10^5$ copies (medium $C_p$ 20.1 ; SD 0.043), (c) $10^4$ copies (medium Cp 23.5; SD 0.029), (d) $10^3$ copies (medium $C_p$ 26.9; SD 0.020), (e) $10^2$ copies (medium $C_p$ 30.4 ; SD 0.2).

Figure 7 comprises 96 real-time amplification curves recorder in 96 different wells of one 384 well plate, each containing $10^4$ copies of the target sequence. The 96 amplification reactions had an average $C_p$-value of 23.7 with a standard deviation of 0.08.

*Sequence information of the Parvovirus B19 (positions of the primers are underlined) SEQ ID NO:1:*

```
1     cagaggttgt gccatttaat gggaagggaa ctaaggctag cataaagttt caaactatgg
61    taaactggct gtgtgaaaac agagtgttta cagaggataa gtggaaacta gttgacttta
121   accagtacac tttactaagc agtagtcaca gtggaagttt tcaaattcaa agtgcactaa
181   aactagcaat ttataaagca actaatttag tgcctactag cgcatttttta ttgcatacag
241   actttgagca ggttatgtgt attaaagaca ataaaattgt taaattgtta ctttgtcaaa
301   actatgaccc cctattggtg gggcagcatg tgttaaagtg gattgataaa aaatgtggca
361   agaaaaatac actgtggttt tatgggccgc caagtacagg aaaaacaaac ttggcaatgg
421   ccattgctaa aagtgttcca gtatatggca tggttaactg gaataatgaa aactttccat
481   ttaatgatgt agcaggaaaa agcttggtgg tctgggatga aggtattatt aagtctacaa
541   ttgtagaagc tgcaaaagct attttaggcg ggcaacccac cagggtagat taaaaaatgc
601   gtggaagtgt agctgtgcct ggagtacctg tggttataac cagcaatggt gacattactt
661   ttgttgtaag cgggaacact acaacaactg tacatgctta agccttaaaa gagcgaatgg
721   taaagttaaa ctttactgta ag
```

*Sequences of PCR primers and probes:*

| | |
|---|---|
| PCR-primer sense (SEQ ID NO:2): | 5'-GGG GCA GCA TGT GTT AAA GTG G-3' |
| PCR-primer antisense (SEQ ID NO:3): | 5'-CCT GCT ACA TCA TTA AAT GGA AAG-3' |
| Acceptor probe (SEQ ID NO:4): | 5'-LCRed640-TTG GCG GCC CAT AAA ACC ACA GTG TAT-phosphate-3' |
| Donor probe (SEQ ID NO:5): | 5'-TGG CCA TTG CCA AGT TTG TTT TTC CTG T-Fluorescein-3' |

*Sequence of amplified fragment:*

```
5'- g gggcagcatg tgttaaagtg gattgataaa aaatgtggca agaaaaatac actgtggttt
    tatgggccgc caagtacagg aaaaacaaac ttggcaatgg ccattgctaa aagtgttcca
    gtatatggca tggttaactg gaataatgaa aactttccat ttaatgatgt agcagg -3'
```

## Claims

**1.** A device for the simultaneous thermocycling of multiple samples comprising:

a) a thermal base (8) formed like a thermal block comprising said multiple samples,
b) at least one heat pump (2),
c) a thermal base (4),
d) a heat sink (5) and
e) a control unit (3) to control said simultaneous thermocycling of multiple samples,
**characterized in that** the thermal base (4) is arranged between and is in thermal contact with said heat sink (5) and said at least one heat pump (2), said at least one heat pump (2) is in thermal contact with said thermal base (8), wherein said thermal base (4) is a vapor chamber device for transporting and distributing heat and wherein said thermal contact is based on a paste having a high thermal conductance, a thermally conductive foil or mechanical force.

**2.** The device according to claim 1, wherein said thermal base (8) comprises recesses (7) disposed to receive said multiple samples.

**3.** The device according to claims 1-2, wherein said at least one heat pump (2) is a thermoelectric device.

**4.** The device according to claims 1 - 3, wherein said thermal base (4) is substantially planar.

**5.** The device according to claim 4, wherein said thermal base (4) is free of recesses.

**6.** The device according to claims 4 - 5, wherein the cross section area of said thermal base (4) is by less than 20 % larger or smaller as the cross section area of said heat sink (5) and wherein the cross section area of said thermal base (4) is larger than the cross section area of said thermal block (1), said cross section areas are in parallel to the respective contact areas.

**7.** The device according to claims 1 - 6, wherein said thermal base is provided with control means (9) to vary the heat conducting properties of said thermal base (4).

**8.** The device according to claims 1 - 7, wherein said control unit (3) is adapted to control the properties of said at least one heat pump (2).

**9.** The device according to claim 8, wherein said control unit (3) is further adapted to control said control means (9) to vary the heat conducting properties of said thermal base (4).

**10.** A device for the simultaneous Thermocycling of multiple samples comprising:

a) a thermal block (1) comprising said multiple samples,
b) at least one heat pump (2),
c) a thermal base (4),
d) a heat sink (5) and
e) a control unit (3) to control said simultaneous Thermocycling of multiple samples,
**characterized in that** the thermal base (4) is arranged between and is in thermal contact with said heat sink (5) and said at least one heat pump (2), said at least one heat pump (2) is in thermal contact with said thermal block (1), wherein said thermal base (4) is a vapor chamber device for transporting and distributing heat and wherein said thermal contact is based on a paste having a high thermal conductance, a thermally conductive foil or mechanical force, wherein the cross section area of said thermal base (4) is larger than the cross section area of said thermal block (1) by at least a factor of 1.5 and said thermal base (4) has the same cross section area as said heat sink (2), said cross section areas are in parallel to the respective contact areas.

**11.** The device according to claim 10, wherein said thermal base (4) is substantially planar.

**12.** The device according to claim 11, wherein said thermal base (4) is free of recesses.

**13.** A method for the simultaneous thermocycling of multiple samples comprising the steps

a) providing a thermal base (8) formed like a thermal block with multiple recesses, at least one heat pump (2), a thermal base (4), a heat sink (5) and a control unit (3),
b) arranging said thermal base (8) with multiple recesses, said at least one heat pump (2), said thermal base (4), and said heat sink (5), wherein

- said heat sink (5) is in thermal contact with said thermal base (4),
- said thermal base (4) is in thermal contact with said at least one heat pump (2) and
- said at least one heat pump (2) is in thermal contact with said thermal base (8),

c) placing said multiple samples within the recesses of said thermal base (8) and
d) performing a thermocycling protocol with said control unit (3),
wherein said thermal contact is based on either a paste having a high thermal conductance, a thermally conductive foil or mechanical force, wherein said thermal base (4), (8) is a vapor chamber device for transporting and distributing heat

**14.** The method according to claim 13, wherein said thermal base (4) is substantially planar.

**15.** The method according to claim 14 wherein said thermal base (4) is free of recesses.

**16.** The method according to claims 13 - 14, wherein the cross section area of said thermal base (4) is by less than 20 % larger or smaller than the cross section area of said heat sink (5) and wherein the cross section area of said thermal base (4) is larger than the cross section area of said thermal base (8), said cross section areas are in parallel to the respective contact areas.

**17.** The method according to claims 13 - 16, wherein said thermocycling protocol is suitable to perform nucleic acid amplifications within said multiple samples.

## Patentansprüche

**1.** Vorrichtung zur gleichzeitigen Temperaturwechselbeanspruchung von mehreren Proben, die Folgendes umfasst:

a) eine thermische Basis (8), die wie ein thermischer Block gebildet ist, der mehrere Proben umfasst,
b) mindestens eine Wärmepumpe (2)
c) eine thermische Basis (4)
d) eine Wärmesenke (5) und
e) eine Steuereinheit (3), um die gleichzeitige Temperaturwechselbeanspruchung von mehreren Proben zu steuern,
**dadurch gekennzeichnet, dass** die thermische Basis (4) zwischen der Wärmesenke (5) und der mindestens einen Wärmepumpe (2) angeordnet ist und mit ihnen in thermischem Kontakt ist, wobei die mindestens eine Wärmepumpe (2) in thermischem Kontakt mit der thermischen Basis (8) ist, wobei die thermische Basis (4) eine Dampfkammervorrichtung zum Befördern und Verteilen von Wärme ist und wobei der thermische Kontakt auf einer Paste mit einer hohen thermischen Leitfähigkeit, einer thermisch leitenden Folie oder mechanischer Kraft beruht.

**2.** Vorrichtung nach Anspruch 1, wobei die thermische Basis (8) Aussparungen (7) umfasst, die angeordnet sind, um die mehreren Proben aufzunehmen.

**3.** Vorrichtung nach den Ansprüchen 1-2, wobei die mindestens eine Wärmepumpe (2) eine thermoelektrische Vorrichtung ist.

**4.** Vorrichtung nach den Ansprüchen 1-3, wobei die thermische Basis (4) im Wesentlichen eben ist.

**5.** Vorrichtung nach Anspruch 4, wobei die thermische Basis (4) frei von Aussparungen ist.

**6.** Vorrichtung nach den Ansprüchen 4-5, wobei die Querschnittsfläche der thermischen Basis (4) um weniger als 20 % größer oder kleiner als die Querschnittsfläche der Wärmesenke (5) ist und wobei die Querschnittsfläche der thermischen Basis (4) größer als die Querschnittsfläche des thermischen Blocks (1) ist, wobei die Querschnittsflächen zu den entsprechenden Kontaktflächen parallel sind.

**7.** Vorrichtung nach den Ansprüchen 1-6, wobei die thermische Basis mit Steuermitteln (9) ausgestattet ist, um die Wärmeleitungseigenschaften der thermischen Basis (4) zu variieren.

**8.** Vorrichtung nach den Ansprüchen 1-7, wobei die Steuereinheit (3) ausgelegt ist, die Eigenschaften der mindestens einen Wärmepumpe (2) zu steuern.

**9.** Vorrichtung nach Anspruch 8, wobei die Steuereinheit (3) ferner ausgelegt ist, die Steuermittel (9) zu steuern, um die Wärmeleitungseigenschaften der thermischen Basis (4) zu variieren.

**10.** Vorrichtung zur gleichzeitigen Temperaturwechselbeanspruchung von mehreren Proben, die Folgendes umfasst:

a) einen thermischen Block (10), der mehrere Proben umfasst,
b) mindestens eine Wärmepumpe (2)
c) eine thermische Basis (4)
d) eine Wärmesenke (5) und
e) eine Steuereinheit (3), um die gleichzeitige Temperaturwechselbeanspruchung von mehreren Proben zu steuern,
**dadurch gekennzeichnet, dass** die thermische Basis (4) zwischen der Wärmesenke (5) und der mindestens einen Wärmepumpe (2) angeordnet ist und mit ihnen in thermischem Kontakt ist, wobei die mindestens eine Wärmepumpe (2) in thermischem Kontakt mit dem thermischen Block (1) ist, wobei die thermische Basis (4) eine Dampfkammervorrichtung zum Befördern und Verteilen von Wärme ist und wobei der thermische Kontakt auf einer Paste mit einer hohen thermischen Leitfähigkeit, einer thermisch leitenden Folie oder mechanischer Kraft beruht, wobei die Querschnittsfläche der thermischen Basis (4) um mindestens einen Faktor 1,5 größer als die Querschnittsfläche des thermischen Blocks (1) ist und die thermische Basis (4) dieselbe Querschnittsfläche wie die Wärmesenke (2) besitzt, wobei die Querschnittsflächen parallel zu den entsprechenden Kontaktflächen sind.

**11.** Vorrichtung nach Anspruch 10, wobei die thermische Basis (4) im Wesentlichen eben ist.

**12.** Vorrichtung nach Anspruch 11, wobei die thermische Basis (4) frei von Aussparungen ist.

**13.** Verfahren zur gleichzeitigen Temperaturwechselbeanspruchung von mehreren Proben, das die folgenden Schritte umfasst:

a) Bereitstellen einer thermischen Basis (8), die wie ein thermischer Block mit mehreren Aussparungen gebildet ist, mindestens einer Wärmepumpe (2), einer thermischen Basis (4), einer Wärmesenke (5) und einer Steuereinheit (3),
b) Anordnen der thermischen Basis (8) mit mehreren Aussparungen, der mindestens einen Wärmepumpe (2), der thermischen Basis (4) und der Wärmesenke (5), wobei

- die Wärmesenke (5) in thermischem Kontakt mit der thermischen Basis (4) ist,
- die thermische Basis (4) in thermischem Kontakt mit der mindestens einen Wärmepumpe (2) ist und
- die mindestens eine Wärmepumpe (2) in thermischem Kontakt mit der thermischen Basis (8) ist,

c) Positionieren der mehreren Proben innerhalb der Aussparungen der thermischen Basis (8) und
d) Ausführen eines Temperaturwechselbeanspruchungs-Protokolls mit der Steuereinheit (3),
wobei der thermische Kontakt entweder auf einer Paste mit einer hohen thermischen Leitfähigkeit, einer thermisch leitenden Folie oder mechanischer Kraft beruht, wobei die thermische Basis (4), (8) eine Dampfkammervorrichtung zum Befördern und Verteilen von Wärme ist.

**14.** Verfahren nach Anspruch 13, wobei die thermische Basis (4) im Wesentlichen eben ist.

**15.** Verfahren nach Anspruch 14, wobei die thermische Basis (4) frei von Aussparungen ist.

**16.** Verfahren nach den Ansprüchen 13-14, wobei die Querschnittsfläche der thermischen Basis (4) um weniger als 20 % größer oder kleiner als die Querschnittsfläche der Wärmesenke (5) ist und wobei die Querschnittsfläche der thermischen Basis (4) größer als die Querschnittsfläche der thermischen Basis (8) ist, wobei die Querschnittsflächen zu den entsprechenden Kontaktflächen parallel sind.

**17.** Verfahren nach den Ansprüchen 13-16, wobei das Temperaturwechselbeanspruchungs-Protokoll geeignet ist, Nukleinsäurevervielfältigungen innerhalb der mehreren Proben auszuführen.

## Revendications

**1.** Dispositif pour le thermocyclage simultané d'échantillons multiples comprenant :

a) une base thermique (8) formée comme un bloc thermique comprenant lesdits échantillons multiples,
b) au moins une pompe à chaleur (2),
c) une base thermique (4),
d) un dissipateur thermique (5) et
e) une unité de commande (3) pour commander ledit thermocyclage simultané d'échantillons multiples,
**caractérisé en ce que** la base thermique (4) est agencée entre et est en contact thermique avec ledit dissipateur thermique (5) et ladite au moins une pompe à chaleur (2), ladite au moins une pompe à chaleur (2) est en contact thermique avec ladite base thermique (8), dans lequel ladite base thermique (4) est un dispositif à chambre de vapeur pour transporter et distribuer une chaleur et dans lequel ledit contact thermique est basé sur une pâte ayant une conductance thermique élevée, une feuille thermo-conductrice ou une force mécanique.

**2.** Dispositif selon la revendication 1, dans lequel ladite base thermique (8) comprend des renfoncements (7) disposés de façon à recevoir lesdits échantillons multiples.

3. Dispositif selon les revendications 1 à 2, dans lequel ladite au moins une pompe à chaleur (2) est un dispositif thermoélectrique.

4. Dispositif selon les revendications 1 à 3, dans lequel ladite base thermique (4) est sensiblement plane.

5. Dispositif selon la revendication 4, dans lequel ladite base thermique (4) est dépourvue de renfoncements.

6. Dispositif selon les revendications 4 à 5, dans lequel l'aire de section transversale de ladite base thermique (4) est de moins de 20% plus grande ou plus petite que l'aire de section transversale dudit dissipateur thermique (5) et dans lequel l'aire de section transversale de ladite base thermique (4) est plus grande que l'aire de section transversale dudit bloc thermique (1), lesdites aires de section transversale sont en parallèle aux zones de contact respectives.

7. Dispositif selon les revendications 1 à 6, dans lequel ladite base thermique est prévue avec un moyen de commande (9) pour faire varier les propriétés de conduction de chaleur de ladite base thermique (4).

8. Dispositif selon les revendications 1 à 7, dans lequel ladite unité de commande (3) est adaptée à commander les propriétés de ladite au moins une pompe à chaleur (2).

9. Dispositif selon la revendication 8, dans lequel ladite unité de commande (3) est en outre adaptée à commander ledit moyen de commande (9) pour faire varier les propriétés de conduction de chaleur de ladite base thermique (4).

10. Dispositif pour le thermocyclage simultané d'échantillons multiples comprenant :

a) un bloc thermique (1) comprenant lesdits échantillons multiples,
b) au moins une pompe à chaleur (2),
c) une base thermique (4),
d) un dissipateur thermique (5) et
e) une unité de commande (3) pour commander ledit thermocyclage simultané d'échantillons multiples,
**caractérisé en ce que** la base thermique (4) est agencée entre et est en contact thermique avec ledit dissipateur thermique (5) et ladite au moins une pompe à chaleur (2), ladite au moins une pompe à chaleur (2) est en contact thermique avec ledit bloc thermique (1), dans lequel ladite base thermique (4) est un dispositif à chambre de vapeur pour transporter et distribuer une chaleur et dans lequel ledit contact thermique est basé sur une pâte ayant une conductance thermique élevée, une feuille thermo-conductrice ou une force mécanique, dans lequel l'aire de section transversale de ladite base thermique (4) est plus grande que l'aire de section transversale dudit bloc thermique (1) d'au moins un facteur de 1,5 et ladite base thermique (4) a la même aire de section transversale que ledit dissipateur thermique (5), lesdites aires de section transversale sont en parallèle aux zones de contact respectives.

11. Dispositif selon la revendication 10, dans lequel ladite base thermique (4) est sensiblement plane.

12. Dispositif selon la revendication 11, dans lequel ladite base thermique (4) est dépourvue de renfoncements.

13. Procédé pour le thermocyclage simultané d'échantillons multiples comprenant les étapes de

a) prévision d'une base thermique (8) formée comme un bloc thermique avec des renfoncements multiples, d'au moins une pompe à chaleur (2), d'une base thermique (4), d'un dissipateur thermique (5) et d'une unité de commande (3),
b) agencement de ladite base thermique (8) avec des renfoncements multiples, de ladite au moins une pompe à chaleur (2), de ladite base thermique (4), et dudit dissipateur thermique (5), dans lequel

- ledit dissipateur thermique (5) est en contact thermique avec ladite base thermique (4),
- ladite base thermique (4) est en contact thermique avec ladite au moins une pompe à chaleur (2) et
- ladite au moins une pompe à chaleur (2) est en contact thermique avec ladite base thermique (8),

c) mise en place desdits échantillons multiples à l'intérieur des renfoncements de ladite base thermique (8) et
d) exécution d'un protocole de thermocyclage avec ladite unité de commande (3),
dans lequel ledit contact thermique est basé sur une pâte ayant une conductance thermique élevée, une feuille thermo-conductrice ou une force mécanique, dans lequel ladite base thermique (4), (8) est un dispositif à chambre de vapeur pour transporter et distribuer une chaleur.

**14.** Procédé selon la revendication 13, dans lequel ladite base thermique (4) est sensiblement plane.

**15.** Procédé selon la revendication 14, dans lequel ladite base thermique (4) est dépourvue de renfoncements.

**16.** Procédé selon les revendications 13 à 14, dans lequel l'aire de section transversale de ladite base thermique (4) est de moins de 20% plus grande ou plus petite que l'aire de section transversale dudit dissipateur thermique (5) et dans lequel l'aire de section transversale de ladite base thermique (4) est plus grande que l'aire de section transversale de ladite base thermique (8), lesdites aires de section transversale sont en parallèle aux zones de contact respectives.

**17.** Procédé selon les revendications 13 à 16, dans lequel ledit protocole de thermocyclage est approprié pour exécuter des amplifications d'acides nucléiques au sein desdits échantillons multiples.

Fig. 1

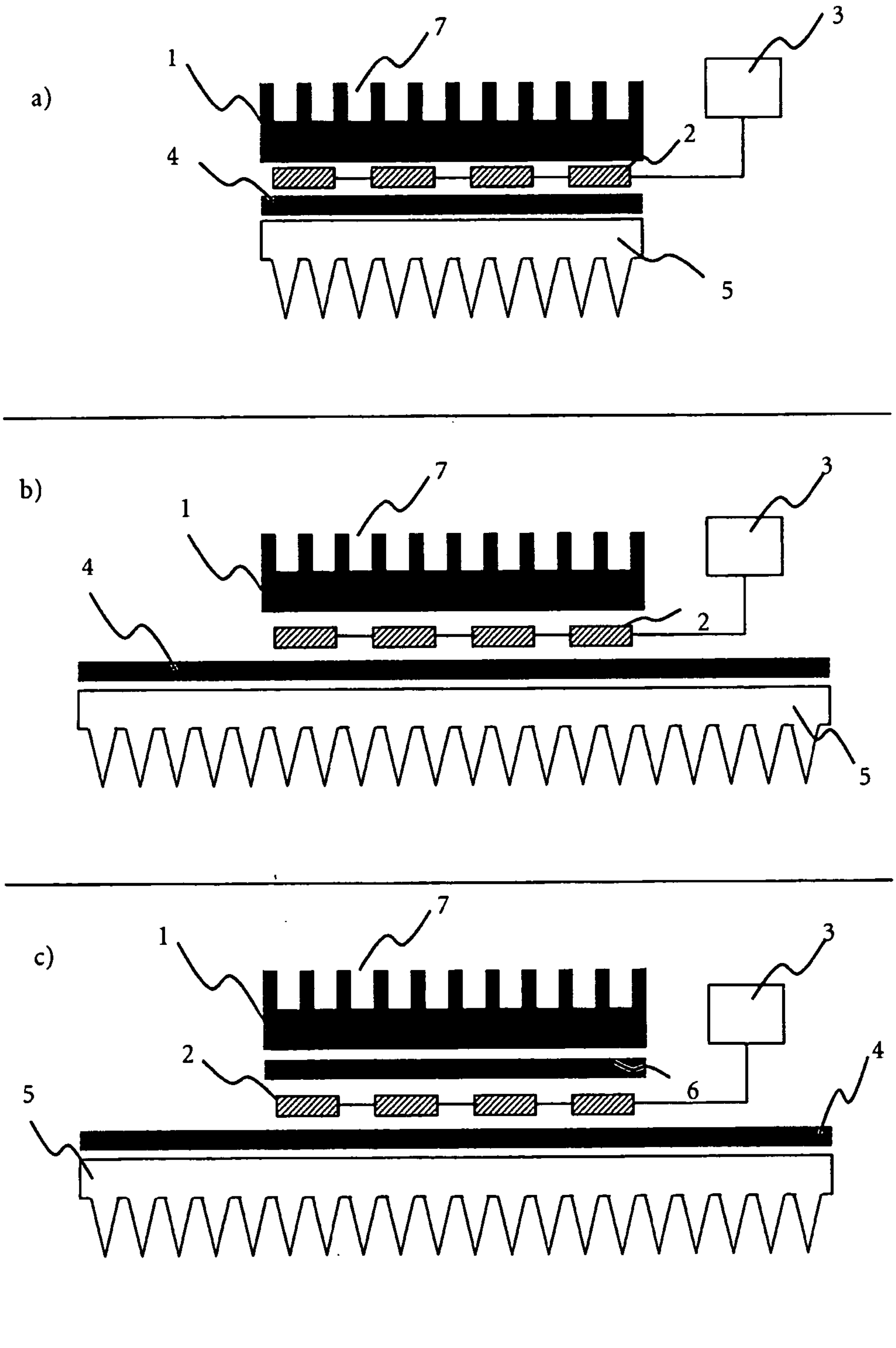

Fig. 1 – continued –

d)

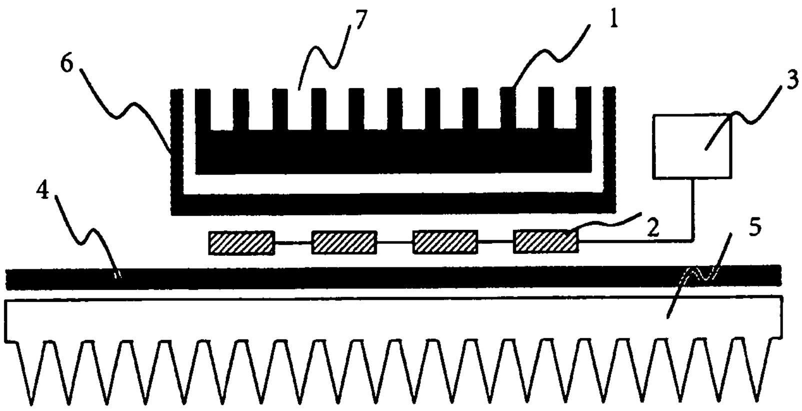


e)

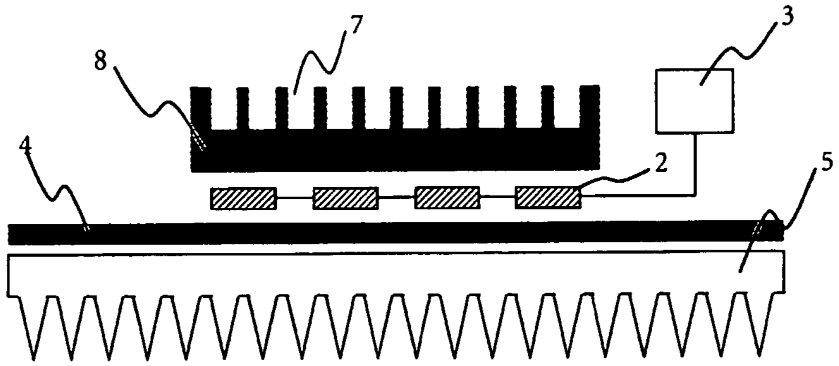


f)

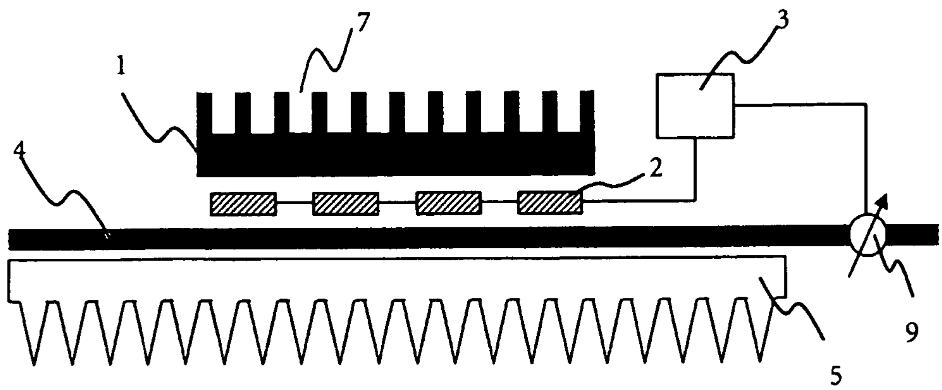

Fig. 2

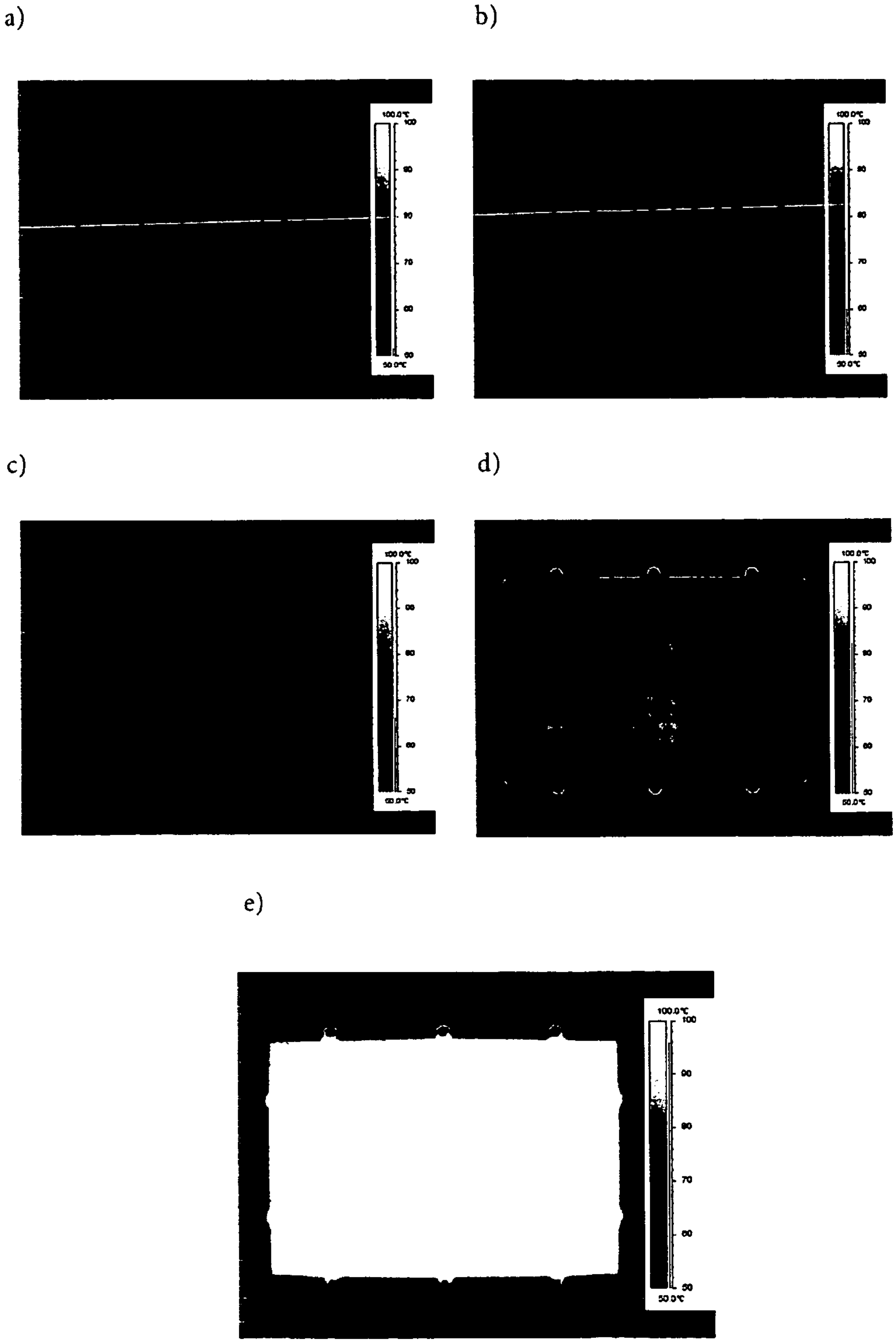

Fig. 3

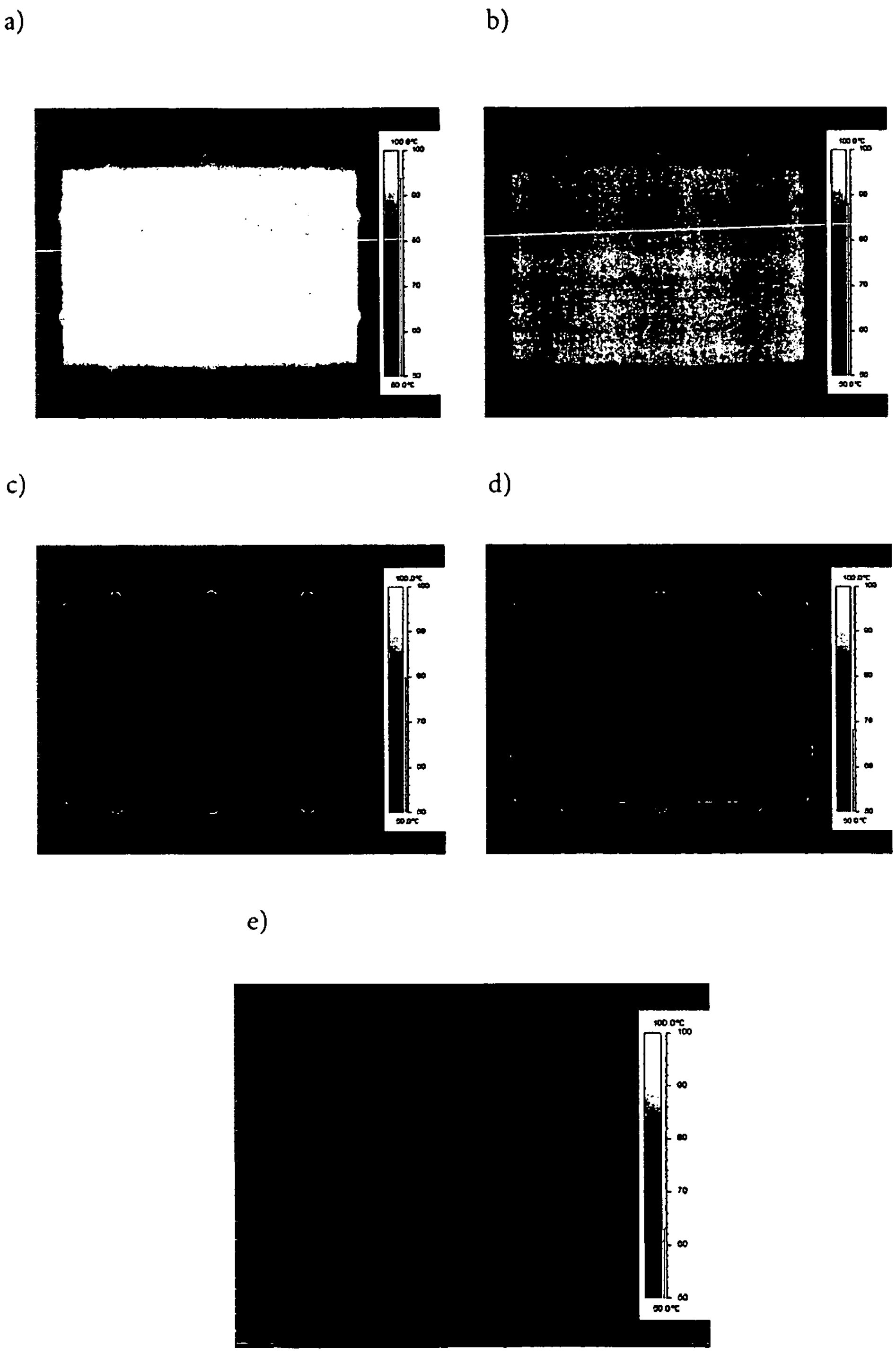

a)

b)

c)

d)

e)

Fig. 4

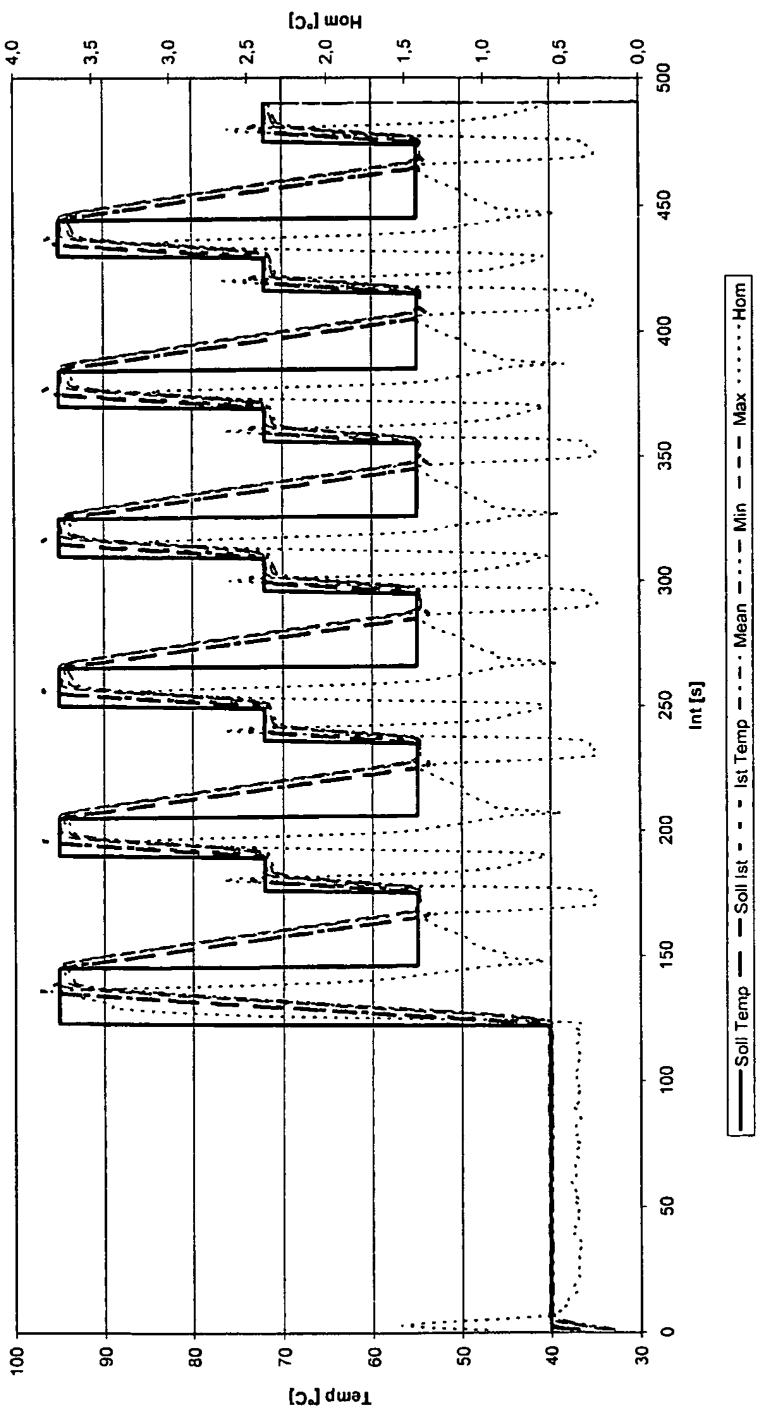

Fig. 5

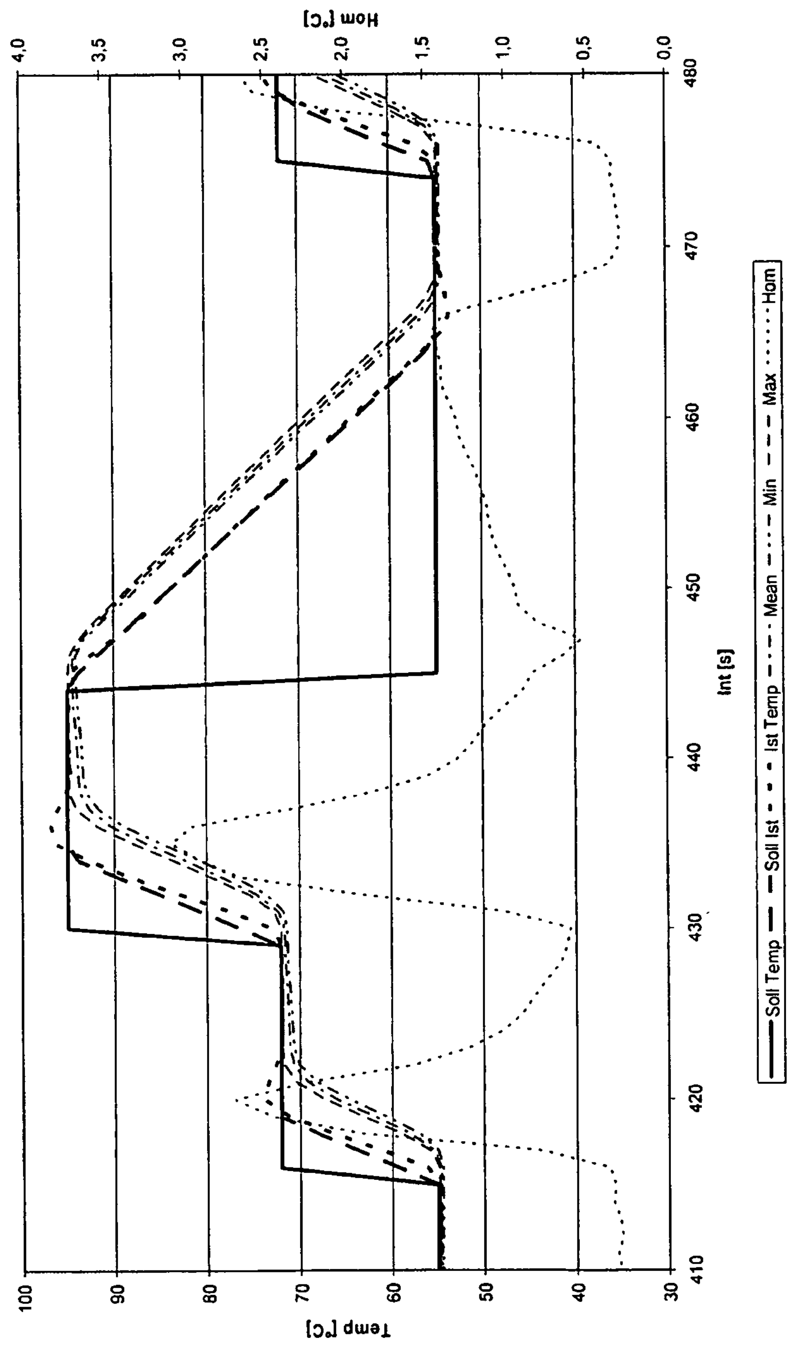

Fig. 6

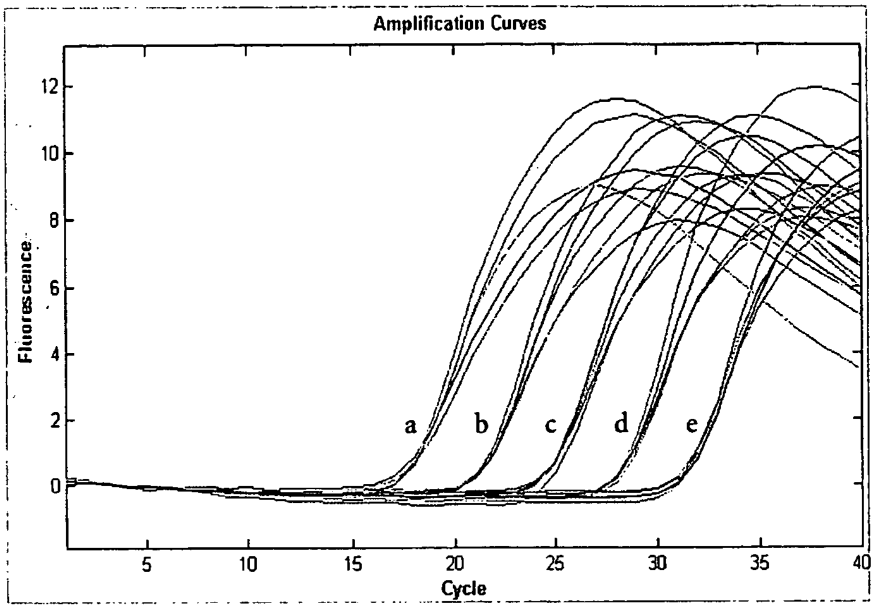
Amplification Curves
Fluorescence
Cycle
a
b
c
d
e
12
10
8
6
4
2
0
5
10
15
20
25
30
35
40

Fig. 7

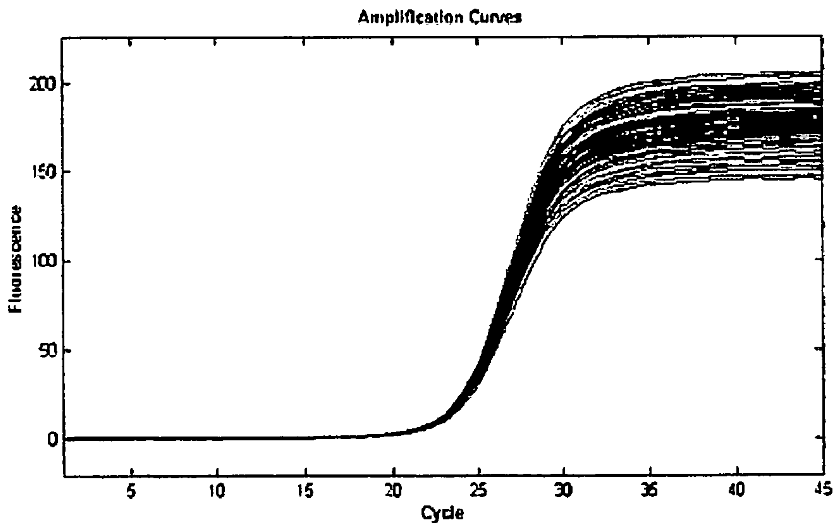
Amplification Curves
Fluorescence
Cycle
200
150
100
50
0
5
10
15
20
25
30
35
40
45

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0151209 A
- US 4950608 A
- US 4387762 A
- US 6256199 B
- US 5161609 A
- US 5819842 A
- WO 0124930 A
- WO 2004105947 A
- US 5417686 A
- EP 05000863 A
- EP 05007267 A


### Non-patent literature cited in the description


- **ROBERT SMYTHE.** *Medical Device & Diagnostic Industry Magazine,* January 1998, 151-157

SEQUENCE LISTING

<110> Roche Diagnostics GmbH F. Hoffmann-La Roche AG

<120> Thermocycling of a block comprising multiple sample

<130> 23080 WO

<150> EP 05007267
<151> 2005-04-04

<160> 5

<170> PatentIn version 3.2

<210> 1
<211> 742
<212> DNA
<213> Parvovirus B19

<400> 1

```
cagaggttgt gccatttaat gggaagggaa ctaaggctag cataaagttt caaactatgg      60
taaactggct gtgtgaaaac agagtgttta cagaggataa gtggaaacta gttgacttta     120
accagtacac tttactaagc agtagtcaca gtggaagttt tcaaattcaa agtgcactaa     180
aactagcaat ttataaagca actaatttag tgcctactag cgcatttttta ttgcatacag    240
actttgagca ggttatgtgt attaaagaca ataaaattgt taaattgtta ctttgtcaaa     300
actatgaccc cctattggtg gggcagcatg tgttaaagtg gattgataaa aaatgtggca     360
agaaaaatac actgtggttt tatgggccgc caagtacagg aaaaacaaac ttggcaatgg     420
ccattgctaa aagtgttcca gtatatggca tggttaactg gaataatgaa aactttccat     480
ttaatgatgt agcaggaaaa agcttggtgg tctgggatga aggtattatt aagtctacaa     540
ttgtagaagc tgcaaaagct attttaggcg ggcaacccac cagggtagat taaaaaatgc     600
gtggaagtgt agctgtgcct ggagtacctg tggttataac cagcaatggt gacattactt     660
ttgttgtaag cgggaacact acaacaactg tacatgctta agccttaaaa gagcgaatgg     720
taaagttaaa ctttactgta ag                                               742
```

<210> 2
<211> 22
<212> DNA
<213> Artificial

<220>
<223> PCR-primer sense

<400> 2
ggggcagcat gtgttaaagt gg 22

<210> 3

<211> 24
<212> DNA
<213> Artificial

<220>
<223> PCR-primer antisense

<400> 3
cctgctacat cattaaatgg aaag 24

<210> 4
<211> 27
<212> DNA
<213> Artificial

<220>
<223> Acceptor probe

<400> 4
ttggcggccc ataaaaccac agtgtat 27

<210> 5
<211> 28
<212> DNA
<213> Artificial

<220>
<223> Donor probe

<400> 5
tggccattgc caagtttgtt tttcctgt 28